# EUROPEAN PATENT APPLICATION

(11) **EP 4 566 511 A1**
(43) Date of publication of application: **11.06.2025**
(21) Application number: 23849699.6
(22) Date of filing: 10.04.2023
(51) Int. Cl.: A61B 1/12, A61B 1/00, G02B 23/24

(54) **CONNECTOR FOR SUBORDINATE WATER-FEEDING MOUTHPIECE OF ENDOSCOPE**

(30) Priority: 02.08.2022 JP 2022123353
(71) Applicant: Ort Medical Co., Ltd., Yokohama-shi, Kanagawa, 222-0033 (JP)
(72) Inventor: KARASAWA Koji, Yokohama-shi, Kanagawa 222-0033 (JP)
(74) Representative: Schön, Christoph
(86) International application number: PCT/JP2023/014537
(87) International publication number: WO 2024/029136

(57) **Abstract**

Provided is an inexpensive connector that prevents occurrence of water leakage in between the connector and an auxiliary water feed mouthpiece. A connector for an auxiliary water feed mouthpiece of an endoscope is to be used to connect a tube unit installed in a water feed device to an auxiliary water feed mouthpiece of an endoscope, which has a flange portion on its outer peripheral surface, and includes a connector main body being made of a synthetic resin and having a cylindrical shape. The connector main body is to be connected to the auxiliary water feed mouthpiece of the endoscope on one end side in a center axis direction of the connector main body, and, when the connector main body is connected to the auxiliary water feed mouthpiece of the endoscope, an end surface of the connector main body on the one end side in the center axis direction of the connector main body is held in a state of being in pressure-contact with an end surface of the flange portion.

## Description

### Technical Field

The present invention relates to a connector for an auxiliary water feed mouthpiece of an endoscope, which is to be connected to an auxiliary water feed mouthpiece provided to an endoscope.

### Background Art

In a medical field, examinations and surgical operations using an endoscope have been widely performed. Such an endoscope has an air feed port, a water feed port, and an auxiliary water feed port, which are formed in a distal end surface of an insertion portion to be inserted into a body cavity of a patient. The auxiliary water feed port allows cleaning of a target site in the body cavity of the patient concurrently with using a treatment instrument through a forceps channel. For example, the endoscope having the auxiliary water feed port has a channel (auxiliary water feed channel) for allowing communication between the auxiliary water feed port and a mouthpiece (auxiliary water feed mouthpiece) (see, for example, Patent Literature 1). The auxiliary water feed port is formed in a nozzle provided at a distal end of the insertion portion. The mouthpiece (auxiliary water feed mouthpiece) is provided to a connecting portion to be connected to a light source device. The auxiliary water feed mouthpiece provided to the connecting portion is a member made of a metal material such as stainless steel. For example, a connector made of the same material as the material of the auxiliary water feed mouthpiece (hereinafter may also be referred to as "connector made of metal") is connected to the auxiliary water feed mouthpiece so that saline from an auxiliary water feed tube unit connected to a water feed device is fed to the auxiliary water feed channel via the connector made of metal.

Incidentally, the above-mentioned connector made of metal includes a cylindrical member and a fixing member. The cylindrical member has one end in a center axis direction to be inserted into the auxiliary water feed mouthpiece and another end in the center axis direction to which the auxiliary water feed tube unit is to be connected. The fixing member is to be tightened and fixed to the auxiliary water feed mouthpiece under a state in which the cylindrical member is inserted. When the connector made of metal is connected to the auxiliary water feed mouthpiece, a tapered surface formed on one end portion of the cylindrical member is brought into pressure-contact with an inner peripheral surface of the auxiliary water feed mouthpiece. In this manner, water leakage in between the connector made of metal and the auxiliary water feed mouthpiece is prevented.

### Citation List

### Patent Literature

[PTL 1] JP 2014-057637 A

### Summary of Invention

### Technical Problem

For example, the endoscope that has been used for an examination or a surgical operation is cleaned and then is subjected to a sterilization treatment as part of infection control. Further, the connector made of metal is mounted to the auxiliary water feed mouthpiece of the endoscope at the time of preparation for an examination or a surgical operation and is then demounted from the auxiliary water feed mouthpiece of the endoscope after the examination or the surgical operation is finished. Repeated actions of mounting and demounting the connector made of metal to and from the auxiliary water feed mouthpiece of the endoscope cause wear of the fixing member or the auxiliary water feed mouthpiece, causing insufficient tightening of the fixing member to the auxiliary water feed mouthpiece. As a result, a pressure-contact state between the tapered surface formed on one end portion of the cylindrical member and a tapered surface in an inner peripheral surface of the auxiliary water feed mouthpiece deteriorates, resulting in water leakage in between the auxiliary water feed mouthpiece and the connector. In this case, the connector made of metal needs to be repaired or replaced, and costs are required to repair or replace the connector made of metal. Thus, there is a demand for an inexpensive connector capable of preventing occurrence of water leakage in between the connector and the auxiliary water feed mouthpiece while the connector is connected to the auxiliary water feed mouthpiece.

The present invention has been made to solve the problem described above, and has an object to provide an inexpensive connector that prevents occurrence of water leakage in between the connector and an auxiliary water feed mouthpiece while the connector is connected to the auxiliary water feed mouthpiece.

### Solution to Problem

In order to solve the above-mentioned problem, according to the present invention, there is provided a connector for an auxiliary water feed mouthpiece of an endoscope, the connector being for use in connecting a tube unit installed in a water feed device to an auxiliary water feed mouthpiece of an endoscope, the auxiliary water feed mouthpiece having a flange portion on an outer peripheral surface of the auxiliary water feed mouthpiece, the connector including a connector main body, which is made of a synthetic resin, and has a cylindrical shape, in which the connector main body is to be connected to the auxiliary water feed mouthpiece of the endoscope on one end side in a center axis direction of the connector main body, and in which, when the connector main body is connected to the auxiliary water feed mouthpiece of the endoscope, an end surface of the connector main body on the one end side in the center axis direction of the connector main body is held in a state of being in pressure-contact with an end surface of the flange portion.

Further, it is preferred that: the connector main body include: a first space, which is defined on another end side in the center axis direction of the connector main body, and allows inflow of a liquid from the tube unit; a second space, which is defined on the one end side in the center axis direction of the connector main body, and allows the liquid flowing into the first space to be fed into the auxiliary water feed mouthpiece to which the connector main body is connected; and a third space, which is arranged between the first space and the second space, and has a smaller sectional area orthogonal to the center axis direction of the connector main body than a sectional area of the first space and a sectional area of the second space; an inner diameter of the second space be set smaller than an outer diameter of the end surface of the flange portion of the auxiliary water feed mouthpiece; and an outer diameter of the connector main body be set larger than the outer diameter of the end surface of the flange portion of the auxiliary water feed mouthpiece.

Further, it is preferred that: the auxiliary water feed mouthpiece have a male thread portion on an outer peripheral surface of a distal end portion of the auxiliary water feed mouthpiece; the connector main body have a female thread portion on an inner peripheral surface of the second space, the female thread portion being threadably engageable with the male thread portion of the auxiliary water feed mouthpiece; and the connector main body be tightened and fixed to the auxiliary water feed mouthpiece under a state in which the male thread portion and the female thread portion are threadably engaged with each other.

Further, it is preferred that the connector main body be configured to allow connection of a check valve fixed to one end of the tube unit to, on a side opposite to one end portion to be connected to the auxiliary water feed mouthpiece of two end portions of the connector main body in the center axis direction of the connector main body, another end portion of the two end portions.

### Advantageous Effects of Invention

According to the present invention, it is possible to provide the inexpensive connector that prevents occurrence of water leakage in between the connector and the auxiliary water feed mouthpiece while the connector is connected to the auxiliary water feed mouthpiece.

### Brief Description of Drawings

FIG. 1 is a view for illustrating one configuration of an endoscope system according to this embodiment.
FIG. 2 is a view for illustrating a configuration of a distal end of a nozzle provided to an insertion portion of an endoscope.
FIG. 3 is a view for illustrating a state before a connector for an auxiliary water feed mouthpiece is mounted to an auxiliary water feed mouthpiece provided to a connecting portion of the endoscope.
FIG. 4 is a sectional view of the auxiliary water feed mouthpiece provided to the connecting portion of the endoscope and the connector for an auxiliary water feed mouthpiece.
FIG. 5 is a partial sectional view for illustrating a state in which a check valve is connected to the connector for an auxiliary water feed mouthpiece that is connected to the auxiliary water feed mouthpiece provided to the connecting portion of the endoscope.

### Description of Embodiments

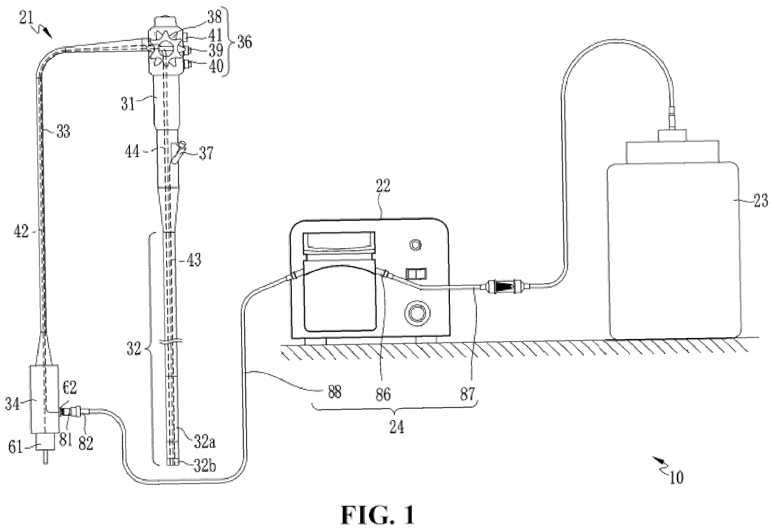

Now, this embodiment is described with reference to the drawings. As illustrated in FIG. 1, an endoscope system 10 includes, for example, an endoscope 21, a water feed device 22, a water feed tank 23, and a pump tube unit 24. The pump tube unit 24 corresponds to a tube unit described in claims.

The endoscope 21 includes an operation handle 31, an endoscope insertion portion 32, a universal tube 33, and a connecting portion 34. The operation handle 31 includes, for example, a plurality of operating portions 36 and a forceps insertion portion 37. The plurality of operating portions 36 include, for example, an ankle knob 38 and various other buttons, such as an air/water feed button 39, a suction button 40, and a shutter button 41. The ankle knob 38 is rotatably operated when a curvable portion 32a of the endoscope insertion portion 32, which is located on a distal end side of the endoscope insertion portion 32, is to be curved. The air/water feed button 39 is operated when an observation port 51 and illumination ports 52 and 53, which are formed at a distal end of a nozzle 32b of the endoscope insertion portion 32, are to be cleaned. The suction button 40 is operated when an unwanted matter in a body cavity (including a liquid that has been used to clean a target site in a body cavity) is to be sucked through a forceps port 55 formed at the distal end of the nozzle 32b of the endoscope insertion portion 32. The shutter button 41 is operated when an image of, for example, the target site in the body cavity is to be picked up with use of an image pickup apparatus (not shown) provided in a distal end of the endoscope insertion portion 32.

A treatment instrument such as forceps is inserted into the forceps insertion portion 37. When the endoscope 21 is not in use, a forceps plug is mounted into the forceps insertion portion 37.

The endoscope insertion portion 32 includes an air feed channel and a water feed channel which are not shown. The endoscope insertion portion 32 also includes a light guide 42, a forceps channel 43, and an auxiliary water feed channel 44. The light guide 42 and the auxiliary water feed channel 44 extend from the endoscope insertion portion 32 to the connecting portion 34 via the universal tube 33. A treatment instrument such as forceps is inserted through the forceps channel 43. Although not shown, a suction channel (not shown) extending to the universal tube 33 and the connecting portion 34 is connected to an upper end portion of the forceps channel 43.

The light guide 42 guides light, which is emitted from a light source device (not shown) connected to the connecting portion 34, to the illumination ports 52 and 53 formed at the distal end of the nozzle 32b of the endoscope insertion portion 32. The forceps channel 43 guides a treatment instrument such as forceps, which is inserted from the forceps insertion portion 37 provided to the operation handle 31, to the forceps port 55 formed at the distal end of the nozzle 32b of the endoscope insertion portion 32. The auxiliary water feed channel 44 guides the liquid fed from the pump tube unit 24 connected to an auxiliary water feed mouthpiece 62 described later to an auxiliary water feed port 56 formed at the distal end of the nozzle 32b of the endoscope insertion portion 32.

The universal tube 33 connects the operation handle 31 and the connecting portion 34 to each other. The universal tube 33 includes inside, for example, the light guide 42, the auxiliary water feed channel 44, and the suction channel (not shown).

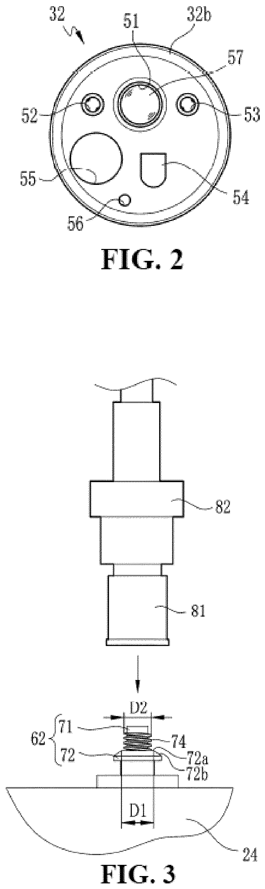

As illustrated in FIG. 2, a distal end surface of the nozzle 32b of the endoscope insertion portion 32 includes, for example, the observation port 51, the illumination ports 52 and 53, an air/water feed nozzle 54, the forceps port 55, and the auxiliary water feed port 56. For a configuration of the nozzle 32b, an example is merely described and illustrated. Types and positions of openings of the nozzle 32b can be appropriately set.

The observation port 51 allows an objective lens 57 of the image pickup apparatus provided in the distal end of the endoscope insertion portion 32 to be exposed therethrough. The objective lens 57 captures an observation image of the target site in the body cavity into the image pickup apparatus. The illumination ports 52 and 53 allow the light guided through the light guide 42 to be emitted as illumination light toward the target site inside the body cavity and the vicinity of the target site. The air/water feed nozzle 54 jets air or a liquid toward the observation port 51 and the illumination ports 52 and 53 so as to clean the observation port 51, the illumination ports 52 and 53, and the vicinity thereof. A distal end of the treatment instrument such as forceps inserted from the forceps insertion portion 37 is exposed and retracted through the forceps port 55. The auxiliary water feed port 56 jets a liquid fed to the auxiliary water feed channel 44 of the endoscope 21 via the pump tube unit 24 through the drive of the water feed device 22.

Returning to FIG. 1, the connecting portion 34 includes, for example, a light-source connecting portion 61, the auxiliary water feed mouthpiece 62, and a mouthpiece for suction (not shown). The light-source connecting portion 61 allows connection to the light source device (not shown). The auxiliary water feed mouthpiece 62 allows connection of the pump tube unit 24.

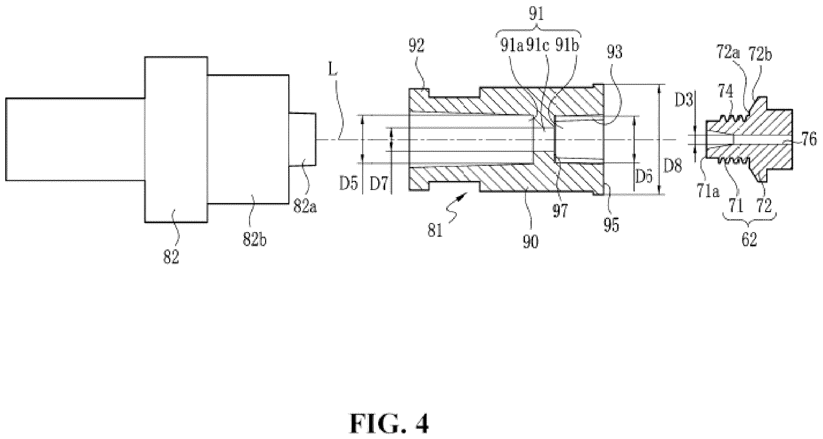

As illustrated in FIG. 3 and FIG. 4, the auxiliary water feed mouthpiece 62 provided to the connecting portion 34 of the endoscope 21 connects to a connector 81 for an auxiliary water feed mouthpiece. A check valve 82, to which one end portion of the pump tube unit 24 is connected, is connected to the connector 81 for an auxiliary water feed mouthpiece. The connector 81 for an auxiliary water feed mouthpiece is hereinafter referred to as "connector 81". A configuration of the connector 81 is described later.

The auxiliary water feed mouthpiece 62 is a cylindrical member made of a metal material, for example, stainless steel, and includes an insertion portion 71 and a flange portion 72. When the connector 81 is connected to the auxiliary water feed mouthpiece 62, the insertion portion 71 is inserted into a second flow passage 91b of the connector 81. The insertion portion 71 has a male thread portion 74 on its outer peripheral surface. When the connector 81 is connected to the auxiliary water feed mouthpiece 62, the male thread portion 74 is threadably engaged with a female thread portion 93 formed on an inner peripheral surface of the connector 81, which faces the second flow passage 91b.

The flange portion 72 is provided so as to hold a mounting portion of a cap (not shown) on the auxiliary water feed mouthpiece 62. The flange portion 72 has an end surface 72a on the insertion portion 71 side of the auxiliary water feed mouthpiece 62 and a tapered surface 72b having a diameter decreased toward the end surface 72a. It is only required that an end surface 95 of the connector 81 be brought into pressure-contact with the end surface 72a of the flange portion 72 when the connector 81 is connected to the flange portion 72. Thus, the flange portion 72 may be a flange portion without the above-mentioned tapered surface 72. Details thereof are described later.

In this case, an outer diameter D1 of the end surface 72a of the flange portion 72 on the insertion portion 71 side is set slightly larger than an outer diameter D2 of the male thread portion 74. Further, the outer diameter D1 of the end surface 72a is larger than a minimum value D6 of an inner diameter of the second flow passage 91b of the connector 81, which is described later, and is smaller than an outer diameter D8 of a connector main body 90 of the connector 81. Thus, when the connector 81 is connected to the auxiliary water feed mouthpiece 62, a peripheral edge portion of one end surface of the connector 81 around the second flow passage 91b is held in a state of being in abutment against the end surface 72a of the flange portion 72. In this case, the outer diameter D2 of the male thread portion 74 is, for example, D2=3.68 mm. Further, the outer diameter D1 of the end surface 72a of the flange portion 72 on the insertion portion 71 side is set larger than the outer diameter D2 of the male thread portion 74 by, for example, about 0.52 mm.

Further, the auxiliary water feed mouthpiece 62 has a flow passage 76 communicating with the auxiliary water feed channel 44. When the connector 81 is connected to the auxiliary water feed mouthpiece 62, the flow passage 76 of the auxiliary water feed mouthpiece 62 and a flow passage 91 of the connector 81 are brought into communication with each other. An inner diameter D3 of the flow passage 76 of the auxiliary water feed mouthpiece 62 is, for example, D3=0.8 mm. In this case, the flow passage 76 corresponds to a mouthpiece-side flow passage described in claims.

Returning to FIG. 1, the water feed device 22 is a device that feeds a liquid stored in the water feed tank 23 toward the endoscope 21 by driving a pump (not shown) built therein. The water feed device 22 is driven by, for example, a pressing operation performed on a foot switch (not shown). When the water feed device 22 is driven, a feed amount of the liquid fed through the pump tube unit 24 is set to, for example, 700 ml/min.

The pump tube unit 24 includes a pump tube 86, a filter-equipped water feed tube 87, and a water feed tube 88. The filter-equipped water feed tube 87 is connected to one end side of the pump tube 86 in its extension direction. The water feed tube 88 is connected to another end side of the pump tube 86 in its extension direction. The filter-equipped water feed tube 87 has, on a side opposite to one end portion connected to the pump tube 86, another end portion connected to the water feed tank 23 in an extension direction of the filter-equipped water feed tube 87. Further, the water feed tube 88 has, on a side opposite to one end portion connected to the pump tube 86, another end portion connected to the check valve 82 in an extension direction of the water feed tube 88. The pump tube 86 is mounted to the water feed device 22. When the water feed device 22 is driven, the liquid stored in the water feed tank 23 is drawn into the pump tube 86 via the filter-equipped water feed tube 87 and the liquid drawn into the pump tube 86 is fed into the water feed tube 88.

In this case, the pump tube 86 used in the pump tube unit 24 has, for example, an inner diameter of 6.6 mm and an outer diameter of 9.7 mm. Further, the filter-equipped water feed tube and the water feed tube have, for example, an inner diameter of 4 mm and an outer diameter of 6.0 mm.

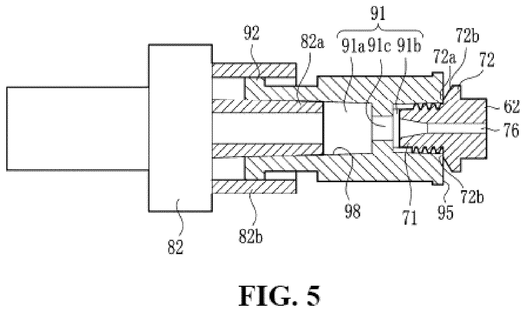

The check valve 82 allows the liquid fed from the pump tube unit 24 to flow into the auxiliary water feed channel 44 of the endoscope 21 while preventing backflow of the liquid from the auxiliary water feed channel 44 of the endoscope 21. The check valve 82 includes a luer tip 82a and a luer lock portion 82b at one end portion in a center axis (L) direction. When the check valve 82 is connected to the connector 81, the luer tip 82a is inserted into a first flow passage 91a of the connector 81 to threadably engage a male thread portion 92 of the connector 81 with a female thread portion (not shown) formed inside the luer lock portion 82b. Then, when the check valve 82 is tightened to the connector 81, an outer peripheral surface of the luer tip 82a is brought into pressure-contact with an inner peripheral surface 98 of the connector 81, which faces the first flow passage 91a (see FIG. 5). The check valve 82 and the connector 81 are individual components. However, the check valve 82 and the connector 81 may be an integrated component.

As described above, the connector 81 is connected to the auxiliary water feed mouthpiece 62. The connector 81 is a single component including the connector main body 90 having a cylindrical shape and being made of, for example, a synthetic resin such as polypropylene, nylon, a fluororesin, an ABS resin, or polycarbonate.

As illustrated in FIG. 4, one end portion of the connector 81 in the center axis (L) direction of the connector 81 functions as a female thread hole, and another end portion thereof in the center axis (L) direction functions as a female-type luer connector portion. The connector 81 has the flow passage 91 extending in the center axis (L) direction. The flow passage 91 includes the first flow passage 91a and the second flow passage 91b in two end portions in the center axis (L) direction of the connector 81, respectively, and a third flow passage 91c. The first flow passage 91a functions as the female-type luer connector portion, and the second flow passage 91b functions as the female thread hole. The third flow passage 91c allows communication between the first flow passage 91a and the second flow passage 91b. In this case, the first flow passage 91a, the second flow passage 91b, and the third flow passage 91c correspond to a first space, a second space, and a third space described in claims, respectively. The second flow passage 91b has a diameter slightly increased from a bottom surface toward one end portion (end surface 95 side) in the center axis (L) direction of the connector 81.

Among these flow passages 91a, 91b, and 91c, a minimum value D5 of an inner diameter of the first flow passage 91a is set to the same value as or slightly larger than the minimum value D6 of the inner diameter of the second flow passage 91b. Further, an inner diameter D7 of the third flow passage 91c is set smaller than the minimum value D5 of the inner diameter of the first flow passage 91a and the minimum value D6 of the inner diameter of the second flow passage 91b. The minimum value D5 of the inner diameter of the first flow passage 91a is, for example, 3.75 mm, and the minimum value D6 of the inner diameter of the second flow passage 91b is, for example, 3.7 mm. The inner diameter D7 of the third flow passage 91c is, for example, 1.8 mm. It is only required that the inner diameter D7 of the third flow passage 91c be smaller than the minimum value D5 of the inner diameter of the first flow passage 91a and the minimum value D6 of the inner diameter of the second flow passage 91b. Thus, it is preferred that the inner diameter D7 of the third flow passage 91c fall within a range of, for example, from 0.8 mm to 3.3 mm.

The connector 81 has the male thread portion 92 on the outer peripheral surface of one end portion in which the first flow passage 91a is defined in the center axis (L) direction of the connector 81. When the check valve 82 is connected to the connector 81, the male thread portion 92 is threadably engaged with the female thread portion (not shown) of the check valve 82.

Further, the connector 81 has the female thread portion 93 on the inner peripheral surface facing the second flow passage 91b. When the connector 81 is connected to the auxiliary water feed mouthpiece 62, the male thread portion 74 of the auxiliary water feed mouthpiece 62 is threadably engaged with the female thread portion 93.

Next, an action of the connector 81 in this embodiment is described. Before an examination or a surgical operation using the endoscope 21 is performed, the connector 81 is tightened and fixed to the auxiliary water feed mouthpiece 62 provided to the connecting portion 34 of the endoscope 21. After the connector 81 is tightened and fixed to the auxiliary water feed mouthpiece 62, a peripheral edge portion continuous with the second flow passage 91b on the end surface 95 of the end portion of two end portions of the connector 81 in the center axis (L) direction of the connector 81, which is to be connected to the auxiliary water feed mouthpiece 62, is held in a state of being in pressure-contact with the end surface 72a of the flange portion 72 of the auxiliary water feed mouthpiece 62. As described above, the outer diameter of the end surface 72a of the flange portion 72 is larger than the minimum value D6 of the inner diameter of the second flow passage 91b and is smaller than the outer diameter D8 of the connector main body 90. Further, the auxiliary water feed mouthpiece 62 is made of a metal material, and the connector main body 90 is made of a synthetic resin. Thus, when the connector 81 is tightened and fixed to the auxiliary water feed mouthpiece 62, the end surface 72a of the flange portion 72 of the auxiliary water feed mouthpiece 62 slightly bites into the end surface 95 of the connector 81 due to a difference in hardness between the auxiliary water feed mouthpiece 62 and the connector 81. A distal end surface 71a of the insertion portion 71 of the auxiliary water feed mouthpiece 62 may be in abutment against a stepped surface 97 between the second flow passage 91b and the third flow passage 91c or may be separate from the stepped surface 97 with a slight gap therebetween.

After the connector 81 is tightened and fixed to the auxiliary water feed mouthpiece 62 provided to the connecting portion 34 of the endoscope 21, the check valve 82 fixed to the pump tube unit 24 is tightened and fixed to another end portion of the connector 81 in the center axis (L) direction of the connector 81. In this manner, the pump tube unit 24 and the endoscope 21 are connected to each other.

After the connector 81 is tightened and fixed to the auxiliary water feed mouthpiece 62, the check valve 82 mounted to one end portion of the water feed tube 88 of the pump tube unit 24 is tightened and fixed to the connector 81. As illustrated in FIG. 3, however, the connector 81 may be tightened and fixed to the auxiliary water feed mouthpiece 62 after the connector 81 is tightened and fixed to the check valve 82 mounted to one end portion of the water feed tube 88 of the pump tube unit 24.

When the water feed device 22 is driven during the examination or the surgical operation using the endoscope 21, the pump of the water feed device 22 is driven to draw the liquid into the pump tube 86 of the pump tube unit 24 and feed the liquid to the water feed tube 88. Then, the liquid stored in the water feed tank 23 is drawn again into the pump tube 86 of the pump tube unit 24 via the filter-equipped water feed tube 87.

While the water feed device 22 is being driven, feeding of the liquid, which has been drawn into the pump tube 86, into the water feed tube 88 and drawing of the liquid stored in the water feed tank 23 into the pump tube 86 are repeatedly performed. In this manner, the liquid stored in the water feed tank 23 is intermittently fed from the pump tube 86 into the water feed tube 88. As described above, when the water feed device 22 is driven, the feed amount of the liquid through the pump tube unit 24 is 700 ml. In this case, a pressure of the liquid fed from the pump tube 86 into the water feed tube 88 is, for example, 0.35 MPa.

The liquid fed from the pump tube 86 into the water feed tube 88 flows to the connector 81 via the check valve 82. The liquid flowing into the connector 81 flows through the flow passage 91 defined in the connector 81 in the order of the first flow passage 91a, the third flow passage 91c, and the second flow passage 91b. In this case, the inner diameter D7 of the third flow passage 91c is smaller than the inner diameter of the first flow passage 91a and the inner diameter of the second flow passage 91b, and hence the third flow passage 91c functions as an orifice. Specifically, a pressure of the liquid flowing into the second flow passage 91b via the third flow passage 91c becomes equal to or lower than a pressure of the liquid flowing through the first flow passage 91a.

As described above, the connector 81 is tightened and fixed to the auxiliary water feed mouthpiece 62. Thus, the liquid flowing into the second flow passage 91b of the connector 81 flows into the flow passage 76 of the auxiliary water feed mouthpiece 62. In this case, the inner diameter D3 of the flow passage 76 of the auxiliary water feed mouthpiece 62 is smaller than the minimum value D6 of the inner dimeter of the second flow passage 91b of the connector 81. Hence, a part of the liquid that has flowed into the second flow passage 91b of the connector 81 stagnates in a space between the second flow passage 91b and the insertion portion 71 of the auxiliary water feed mouthpiece 62. In this case, the end surface 95 of the connector 81 in the center axis (L) direction is in pressure-contact with the end surface 72a of the flange portion 72 of the auxiliary water feed mouthpiece 62 to thereby prevent leakage of the liquid in between the end surfaces.

After the examination or the surgical operation using the endoscope 21 is finished, the connector 81 is demounted from the auxiliary water feed mouthpiece 62 and is then disposed of together with the check valve 82 and the pump tube unit 24 to which the check valve 82 is connected.

### <Effects>

The connector 81 for an auxiliary water feed mouthpiece according to this embodiment is the connector 81 for an auxiliary water feed mouthpiece to be used to connect the pump tube unit 24 installed in the water feed device 22 to the auxiliary water feed mouthpiece 62 of the endoscope 21, which has the flange portion 72 on its outer peripheral surface. The connector 81 for an auxiliary water feed mouthpiece includes the connector main body 90 being made of a synthetic resin and having a cylindrical shape. The connector main body 90 is to be connected to the auxiliary water feed mouthpiece 62 of the endoscope 21 on one end side in the center axis (L) direction of the connector main body 90. In addition, when the connector main body 90 is connected to the auxiliary water feed mouthpiece 62 of the endoscope 21, the end surface 95 of the connector main body 90 on one end side in the center axis (L) direction is held in a state of being in pressure-contact with the end surface 72a of the flange portion 72.

With the configuration described above, when the connector 81 for an auxiliary water feed mouthpiece is connected to the auxiliary water feed mouthpiece 62 of the endoscope 21, the end surface 95 of the connector main body 90 is brought into pressure-contact with the end surface 72a of the flange portion 72. Thus, the connector 81 for an auxiliary water feed mouthpiece and the auxiliary water feed mouthpiece 62 of the endoscope 21 are held in a liquid-tight manner. Hence, while the liquid is being fed by driving the water feed device 22, occurrence of water leakage in between the connector 81 for an auxiliary water feed mouthpiece and the auxiliary water feed mouthpiece 62 of the endoscope 21 can be prevented. Further, the connector 81 for an auxiliary water feed mouthpiece is formed as a single member made of a synthetic resin. As a result, the inexpensive connector having a simple structure can be provided. Further, such a connector is a disposable product that can be disposed of together with, for example, the pump tube unit 24 after an examination or a surgical operation using the endoscope on a target site is performed.

Further, the connector main body 90 includes the first flow passage 91a, the second flow passage 91b, and the third flow passage 91c. The first flow passage 91a is defined on another end side in the center axis (L) direction of the connector main body 90 and allows inflow of the liquid from the pump tube unit 24. The second flow passage 91b is defined on one end side in the center axis (L) direction of the connector main body 90 and allows the liquid to be fed into the auxiliary water feed mouthpiece 62 to which the connector main body 90 is connected. The third flow passage 91c is arranged between the first flow passage 91a and the second flow passage 91b, and has a sectional area orthogonal to the center axis (L) direction of the connector main body 90 being smaller than those of the first flow passage 91a and the second flow passage 91b. The inner diameter of the first flow passage 91a is set smaller than the outer diameter of the end surface 72a of the flange portion 72 of the auxiliary water feed mouthpiece 62. The outer diameter of the connector main body 90 is set larger than the outer diameter of the end surface 72a of the flange portion 72 of the auxiliary water feed mouthpiece 62.

With the configuration described above, the liquid fed through the pump tube unit 24 by the water feed device 22 flows in the order of the first flow passage 91a, the third flow passage 91c, and the second flow passage 91b of the connector 81. In this case, the third flow passage 91c has a sectional area smaller than that of the first flow passage 91a, and thus functions as an orifice to hold down the pressure of the liquid flowing into the second flow passage 91b via the third flow passage 91b. Further, a part of the liquid flowing through the second flow passage 91b flows into the flow passage 76 of the auxiliary water feed mouthpiece 62, and the remaining liquid stagnates in the space between the second flow passage 91b and the auxiliary water feed mouthpiece 62. The peripheral edge portion of the end surface 95 around the second flow passage 91b in the center axis (L) direction of the connector main body 90 is brought into pressure-contact with the flange portion 72 of the auxiliary water feed mouthpiece 62. In some cases, the end surface 72a of the flange portion 72 of the auxiliary water feed mouthpiece 62 is held in a state of biting into the end surface 95 of the connector main body 90. Thus, a liquid-tight state is ensured between the end surfaces 72a and 95 to thereby prevent occurrence of water leakage.

Further, the auxiliary water feed mouthpiece 62 has the male thread portion 74 on the outer peripheral surface of its distal end portion. The connector main body 90 has the female thread portion 93 on the inner peripheral surface of the second flow passage 91b, which is to be threadably engaged with the male thread portion 74 formed on the auxiliary water feed mouthpiece 62. When the connector main body 90 is tightened to the auxiliary water feed mouthpiece 62 under a state in which the male thread portion 74 and the female thread portion 93 are threadably engaged, the connector main body 90 is connected to the auxiliary water feed mouthpiece 62.

With the configuration described above, the connector main body 90 can easily be mounted to the auxiliary water feed mouthpiece 62 without fixing means for fixing the connector main body 90 to the auxiliary water feed mouthpiece 62.

Further, the connector main body 90 allows the connection of the check valve 82 fixed to one end of the pump tube unit 24 to, on the side opposite to one end portion to be connected to the auxiliary water feed mouthpiece 62 of two end portions of the connector main body 90 in the center axis (L) direction of the connector main body 90, another end portion of the two end portions.

With the configuration described above, occurrence of leakage of the liquid in between the check valve 82 and the connector 81 can be prevented.

Further, after the connector 81 is connected to the auxiliary water feed mouthpiece 62, occurrence of water leakage at the connection to the auxiliary water feed mouthpiece 62 can be prevented without an additional configuration as a configuration on the side of the auxiliary water feed mouthpiece 62 that is in pressure-contact with the end surface 95 of the connector 81.

### Reference Signs List

- 21: endoscope
- 24: pump tube unit
- 44: auxiliary water feed channel
- 62: auxiliary water feed mouthpiece
- 71: insertion portion
- 72: flange portion
- 72a: end surface
- 76: flow passage
- 81: for auxiliary water feed mouthpiece
- 86: pump tube
- 90: connector main body
- 91: flow passage
- 91a: first flow passage
- 91b: second flow passage
- 91c: third flow passage
- 95: end surface

## Claims

1. A connector for an auxiliary water feed mouthpiece, the connector being for use in connecting a tube unit installed in a water feed device to an auxiliary water feed mouthpiece of an endoscope, the auxiliary water feed mouthpiece having a flange portion on an outer peripheral surface of the auxiliary water feed mouthpiece,
the connector comprising a connector main body, which is made of a synthetic resin, and has a cylindrical shape,
wherein the connector main body is to be connected to the auxiliary water feed mouthpiece of the endoscope on one end side in a center axis direction of the connector main body, and
wherein, when the connector main body is connected to the auxiliary water feed mouthpiece of the endoscope, an end surface of the connector main body on the one end side in the center axis direction of the connector main body is held in a state of being in pressure-contact with an end surface of the flange portion.

2. The connector for an auxiliary water feed mouthpiece according to claim 1,
wherein the connector main body includes:
a first space, which is defined on another end side in the center axis direction of the connector main body, and allows inflow of a liquid from the tube unit;
a second space, which is defined on the one end side in the center axis direction of the connector main body, and allows the liquid flowing into the first space to be fed into the auxiliary water feed mouthpiece to which the connector main body is connected; and
a third space, which is arranged between the first space and the second space, and has a smaller sectional area orthogonal to the center axis direction of the connector main body than a sectional area of the first space and a sectional area of the second space,
wherein an inner diameter of the second space is set smaller than an outer diameter of the end surface of the flange portion of the auxiliary water feed mouthpiece, and
wherein an outer diameter of the connector main body is set larger than the outer diameter of the end surface of the flange portion of the auxiliary water feed mouthpiece.

3. The connector for an auxiliary water feed mouthpiece according to claim 2,
wherein the auxiliary water feed mouthpiece has a male thread portion on an outer peripheral surface of a distal end portion of the auxiliary water feed mouthpiece,
wherein the connector main body has a female thread portion on an inner peripheral surface of the second space, the female thread portion being threadably engageable with the male thread portion of the auxiliary water feed mouthpiece, and
wherein the connector main body is tightened and fixed to the auxiliary water feed mouthpiece under a state in which the male thread portion and the female thread portion are threadably engaged with each other.

4. The connector for an auxiliary water feed mouthpiece according to claim 2, wherein the connector main body is configured to allow connection of a check valve fixed to one end of the tube unit to, on a side opposite to one end portion to be connected to the auxiliary water feed mouthpiece of two end portions of the connector main body in the center axis direction of the connector main body, another end portion of the two end portions.
